(19) Europäisches Patentamt
European Patent Office
Office européen des brevets

(11) **EP 2 755 665 B1**

(12) **EUROPEAN PATENT SPECIFICATION**

(45) Date of publication and mention of the grant of the patent:
**25.10.2017 Bulletin 2017/43**

(21) Application number: **12772068.8**

(22) Date of filing: **12.09.2012**

(51) Int Cl.:
*A61K 33/00* [(2006.01)]     *A61K 9/16* [(2006.01)]
*A61P 35/00* [(2006.01)]     *A61P 31/10* [(2006.01)]
*A61P 31/04* [(2006.01)]     *A61K 9/51* [(2006.01)]
*A61K 51/12* [(2006.01)]     *A61K 33/24* [(2006.01)]
*A61N 5/10* [(2006.01)]

(86) International application number:
**PCT/EP2012/003825**

(87) International publication number:
**WO 2013/037487 (21.03.2013 Gazette 2013/12)**

(54) **A PARTICULATE SYSTEM FOR USE IN DIMINISHING CELL GROWTH/INDUCING CELL KILLING**

PARTIKELSYSTEM ENTHALTEND LANTHANIDE ZUR VERWENDUNG BEIM VERRRINGERN DES ZELLWACHSTUMS/INDUZIEREN DER ZELLABTÖTUNG

SYSTÈME À PARTICULES COMPRENANT DES LANTHANIDES POUR DIMINUER LA CROISSANCE CELLULAIRE/INDUCTION D'ÉLIMINATION DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2011 EP 11007401**

(43) Date of publication of application:
**23.07.2014 Bulletin 2014/30**

(73) Proprietors:
• **Johannes-Gutenberg-Universität Mainz**
  **55122 Mainz (DE)**
• **Universitätsmedizin der Johannes Gutenberg**
  **55131 Mainz (DE)**

(72) Inventors:
• **LANGGUTH, Peter**
  **63599 Biebergemünd (DE)**
• **BUCH, Karl**
  **55127 Mainz (DE)**
• **NAWROTH, Thomas**
  **55450 Langenlonsheim (DE)**
• **SCHMIDBERGER, Heinz**
  **55270 Essenheim (DE)**

(74) Representative: **Patentanwälte Dr. Keller, Schwertfeger**
  **Westring 17**
  **76829 Landau (DE)**

(56) References cited:
**WO-A2-2009/121631     US-A1- 2006 204 445**
**US-B2- 6 770 020**

## Description

## Technical Field:

The finding of appropriate therapeutic treatments of diseases such as bacterial or fungal infections, or cancer is still a major task in the field of medicine and pharmacology. Different approaches have been established over time to be utilized in the treatment of these diseases or to minimize their symptoms. In Europe nearly every third man suffers from cancer during the course of his life. Upon diagnosis of cancer, the survival rate within a term of five years is approximately 55 %. In Germany, roughly 400,000 new cases of patients that suffer of cancer are accounted per year. The most frequent type of cancers among the human population is breast cancer, intestinal cancer and lung cancer. These types of cancer are main targets for different medical treatment approaches. Cancer treatments generally include resections of tumor tissue, chemotherapy with cytostatics and angiogenesis inhibitors. In addition, irradiation therapy is supplied in combination with the application of radio pharmaceuticals, X-rays, thermionic irradiation and neutron irradiation.

One problem associated with the application of irradiation can be seen in the high doses required in order to diminish cell growth of treated cancer cells. The high doses of irradiation however cause a number of severe side effects with unpleasant outcome for the patient. One major problem of such irradiation treatment is that not only the cancer tissue is affected by irradiation but also surrounding healthy tissue. It is therefore an aim in radiology to decrease the irradiation doses required for treatment of diseases. Irradiation enhancers were found to maximize the irradiation effects, and thereby minimizing the doses required for diminishing cell growth. Such enhancers are able to achieve a reduction of the irradiation doses utilized for the treatment of target cells.

## Background Art:

Lanthanide compounds and their uses for MRT and other applications have been extensively discussed (Caravan P., Ellison J.J., McMurry T.J., Lauffer R.B. (1999) Chem. Rev. 99, 2293-2352 "Gadolinium(III) Cheleates as MRI Contrast Agents: Structure, Dynamics, and Applications"; Wiener E.C., Konda S., Shadron A., Brechbiel M., Gansow O. (1997) Invest. Radiol. 32, 748-54 "Targeting dendrimer-chelates to tumors and tumor cells expressing the high-affinity folate receptor").

US 6,770,020 B2 describes a method of using gadolinium-containing compounds as agents for neutron capture therapy to treat neoplastic cell growth. The subject is exposed to a gadolinium-containing compound for a time sufficient to allow the compound to accumulate in neoplastic cells. The subject is then exposed to a thermal and/or epithermal neutron flux, thereby initiating a neutron capture reaction in the gadolinium atoms that results in specific death of neoplastic cells.

US 5,888,997 describes irradiation sensitizers and the use of texaphyrins for irradiation sensitization and other conditions for which X-ray irradiation has proven to be therapeutically effective.

EP 01 292 298 B describes halogen compounds for use in a photo therapeutic treatment of a disease. The compounds are used for increasing the efficiency of a radiation therapy. US 6,040,432 describes metal complexes of DTPA derivatives suitable for use in diagnosis and therapy. Heavy elements were used in NMR/MRT diagnostic and as irradiation therapeutics.

T. Nawroth, et al., SRMS 4, Conference "Synchrotron Irradiation in Material Sciences", Grenoble, August 23-25, 2004, describes magnetic liposomes and trapping target hollow magnetic particles for biomedical applications. The method described is used for imaging, and neutron and photodynamic X-ray therapy of cancer. WO2009/121631 A2 describes a polymer-based nanoparticle, a linker comprising a first portion non-covalently anchored to said nanoparticle, and one or more targeting agents for delivery to lung cancer cells, which comprise one or more soluble lanthanide compounds such as Erbium-169, Samarium-153, Yttrium-90 embedded in a solid biodegradable polymer particle. Preferred biocompartible polymers are polyesters such as polyhydroxybutyric acid, polyhydroxyvaleric acid, polycaprolactone, polycyanoacrylate, polycarbonate, polylactide (PLA), poly (lactideco-glycolide), polylactic (also termed polylactide), polyglycolic, acid (also termed polyglycolide), apolylactic- polyglycolic acid. The nanoparticles require specific ligands such as monoclonal antibodies for targeting, wherein coupling is attained by covalent binding of the antibody molecule to the particle surface. US 6,770,020 B2 describes another method of using gadolinium-containing compounds as agents in the treatment of neoplastic cell growth. The subject is exposed to a gadolinium-containing compound for a time sufficient to allow the compound to accumulate in neoplastic cells. The subject is then exposed to a termal and/or epitermal neutron flex, thereby initiating a neutron capture reaction in the gadolinium atoms that results in specific death of neoplastic cells. Although the systems and methods described above may show some effects in killing cancer cells, there is a need to increase the efficiency in the treatment of cancer using lanthanide compounds-containing particles.

## Disclosure of Invention:

Against this background, it is object of the present invention to provide an improved delivery system which is based on a selection of lanthanide compounds embedded into nanoparticles for diminishing growth of target cells, in which the molecular interaction between the lanthanide compound and the structures of the polymer are improved in order to provide high efficiency in the uptake of the lanthanide compounds by the target cells.

This object is solved by a particulate system with the technical features of claim 1. The sub claims relate to preferred embodiments of the present invention.

The present invention provides polymer particles that comprise one or more lanthanide compounds embedded in a solid biodegradable polymer particle, wherein the lanthanide compounds of the polymer particles have a photon energy that is greater than 38 keV and a K absorption edge Z that is greater than 56.

The use of heavy metal lanthanide compounds with a photon energy > 38 keV and a K absorption edge Z > 56 in the particulate system of the invention results in an enhancement of radiation by increasing the radiation absorption diameter due to photo electrical absorption of electrons at the K layer. The radiation of the lanthanide particles of the invention will deeply enter the tissue, in particular cancer tissue, and thus will be able to reach the localisation of the tumour. At the same time, severe burns of the surrounding non-target tissue will be avoided. Contrary to other methods, the methods according to the present invention do not result in a higher sensitisation of cells (chemical sensitizer effect) but apply radiation enhancement. In addition, radioactive or toxic effects are avoided by the methods of the invention.

Preferred heavy metal lanthanide compounds having > 38 keV and a K absorption edge Z > 56 are compounds that are stable isotopes or long-term isotopes with a half-life of more than $10^{10}$ years. Preferred lanthanide compounds have a K absorption edge Z between 57 and 83 and include lanthanide compounds ranging from lanthanide up to bismuth and are non-radioactive.

In one embodiment the polymer particles loaded with one or more lanthanide compounds are provided in freeze-dried form, preferably in the form of freeze-dried powder. Surprisingly, the particulate system in combination with freeze-drying results in an increased efficiency in killing cancer cells as compared to non-modified polymer particles, which are provided in aqueous suspension.

In another embodiment, the polymer particles of the invention are provided in modified, stabilised form in suspension. In order to obtain such stable polymer particles, the surface of the polymer are stabilized by detergents or stabilizers.

The inventors of the particulate system according to the present invention discovered that embedding lanthanide compounds having photon energy that is greater than 38 keV and K absorption edge Z that is greater than 56 into biodegradable polymer particles is highly efficient for killing cancer cells.

The particulate system according to the present invention preferably comprises one or more water soluble lanthanide compounds (including their salts) that are embedded in a solid biodegradable polymer particle for delivery to the target cells. Preferred polymers used for embedding the lanthanide compound or a mixture thereof are polycarbonic acids, polylactic acids, polyglycolic acids, polypeptides or combinations thereof.

The lanthanide compound is preferably selected from the group consisting of lanthanum, cerium, praseodymium, neodymium, promethium, samarium, europium, gadolinium, erbium, dysprosium, holmium, erbium, thulium, ytterbium, lutetium, scandium, yttrium, hafnium iridium, platin, gold, bismuth and their salts. The use of lanthanide salts, preferably acetate salts, is preferred.

Lanthanide compounds that show a characteristic irradiation pattern upon excitation are suitably detectable by well known analysis methods in the art. In one embodiment, erbium acetate is a preferred lanthanide compound to be packed in a solid, freeze-dried biodegradable polymer particle. In an alternative embodiment gadolinium acetate is preferred. It is further possible to combine one or more lanthanide compounds or their salts with other types of irradiation enhancers (e.g. cisplatin, resveratrol, hydroxychalcone, roscovitine, amrubicine, amrubicinol) or even cytostatics such as doxorubicine or Paclitaxel in the freeze-dried polymer particles of the invention.

The polymer of the particulate system is preferably selected from the group consisting of any optically active (D-/L-/DL-) forms of poly(glycolic acid) (PGA), poly(lactic acid) (PLA), poly (lactide-co-glycolide) copolymers (PLGA), polydioxanone (PDS), polyacrylates, polyketales, polycyanoacrylates, polyorthoesters, polyacetates, poly(ε caprolactone), polyphosphozene, polycarbonates, polypeptides, polyiminocarbonates, poly(β-hydroxyester).

Poly(glycolic acid) (PGA), poly(lactic acid) (PLA) and their copolymers are preferred biodegradable polymers of the particulate system according to the present invention. These polymers degrade in the body by hydrolysis of the ester backbone to non-harmful and non-toxic compounds. The degradation products are either excreted by the kidneys or eliminated as carbon dioxide and water through well-known biochemical pathways. The polymers PGA, PLA, PLGA and PDS as well as their copolymers can be used in all optically active forms or as part of a racemic mixture of their active L- and D-forms.

The life-time of polymers within the human or animal body and therefore their effectiveness can be controlled by selecting different appropriate end-groups. Preferably, the polymer utilized in the particulate system of the invention has either a free carboxylic acid end group, an ester terminated end group or an alkyl ester end group. Polymers kept with ester terminated and alkyl ester groups typically show longer degradation life times than the free carboxylic analogues. One preferred polymer of the invention is poly(D,L-lactide-co-glycolide) with a free carboxylic acid end group.

The packaging of lanthanides/lanthanide salt compounds in solid biodegradable polymer particles surprisingly results in a rather high enrichment of the irradiation enhancer within the particle and hence high delivery doses to the target cells. The freeze-dried biodegradable polymer particles are superior in their efficiency to non-modified particles in aqueous suspensions. This may be explained by the formation of solid bounds between the nanoparticles in suspension. As a result, agglomerates are formed, which are poorer absorbed by the cells as compared to freeze-dried particles. The inventors further showed by electron microscopy that the formation of solid bounds between the particles is essentially completed upon storage of the samples over night. By contrast, only little or no solid bounds could be observed using freeze-dried particles. Therefore, the uptake of a lanthanide compound of the invention is a significantly increased using freeze-dried particles.

The efficiency of polymer particles in suspension can be increased, however, by modifying the surface of the particles using detergents or other stabilizers. Such treated particles also show increased efficiency.

The use of lanthanide compounds in form of their acetate salts is preferred since there appears to be an unexpected and surprising molecular interaction between chemical residues of the acetate salt and structures of the polymer. The polymer particles provide a high efficiency in the uptake of the lanthanide compounds by the target cells. The polymer particle of the invention is thus a suitable means to deliver the lanthanide compounds to the target cell (e.g. bacterial, fungal or cancer cell).

One major advantage of the invention is that the particulate system uses polymer particles that are biodegradable. The use of biodegradable polymers avoids an unwanted accumulation of polymer compounds within the treated tissue, in particular in the human or animal body. The biodegradable polymer used in the particulate system of the invention will be physiologically degraded after a certain time.

The particulate system of the invention, consisting of lanthanide compounds embedded in solid biodegradable polymers is preferably produced by solvent evaporation. A defined amount of polymer is diluted in dichloromethane. A lanthanide salt (e.g. erbium acetate) is diluted at high concentration in water resulting in an aqueous phase. The aqueous phase is emulsified within the oily phase of the polymer fraction by treatment with an ultrasonic stirrer on ice. The resulting O/W emulsion is transferred into a W/O/W emulsion by addition of approximately 2.5 x vol of a 1 % aqueous solution of polyvinyl alcohol with an average molecule mass of 72,000 g/mol. A subsequent ultrasonic treatment is followed. The resulting emulsion is stirred slowly in 3 x vol of water, preferably in a round-bottomed flask on a magnetic stirrer at low pressure (approximately 500 mbar) for several hours. Following incubation, the solvent dichloromethane is allowed to enter into the aqueous phase and subsequently into the gas phase. The evaporation of the solvent results in a hardening of the polymer particles and their separation. Upon evaporation of the solvent, the size of the produced particles can be controlled by dynamic light scattering (DLS).

The invention further relates to a method for diminishing cell growth, comprising the steps of exposing cells to a particulate system, comprising one or more water soluble lanthanide compounds as irradiation enhancer, wherein the lanthanide compounds are embedded in a biodegradable polymer particle as carrier, the polymer being selected from the group consisting of polycarbonic acids, polylactic acids, polyglycolic acids, polypeptides or combinations thereof, freeze-drying the particles loaded with one or more lanthanide compounds and exposing the cells that are treated with the particulate system to irradiation at a wave length that results in an excitation of the lanthanide compound(s). The method can be used both for in-vitro and in-vivo treatments. The carrier systems and the methods according to the invention can be used both for therapeutic and diagnostic purposes.

Depending on the lanthanide used, the irradiation dose for treating the target cells is at least 4 Gy. A suitable pre-incubation time for the irradiation treatment of the target cells that were exposed to lanthanide loaded polymer particles is at least 24 h.

The invention further relates to a method for producing particles, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof by incubating the polymer with the lanthanide compound in a suitable solvent solution, emulsifying the polymer/lanthanide mixture and applying solvent evaporation to hardening the particles. The invention also relates to a pharmaceutical composition, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof for use in the treatment of a disease.

In a preferred embodiment, the disease is a bacterial or fungal infection or cancer. The systems and methods according to the invention can be both applied to prokaryotic and eukaryotic cells. Pathological diseases that are caused by a bacterial or fungal infection are based on pathogenic bacteria or fungi. Both bacterial and fungal cells can be exposed to the particulate system according to the invention. Cell growth is inhibited or reduced in these cells by delivering the lanthanide compounds by the polymer carrier to the respective target cells. The systems and methods according to the invention are also suitable for treatment of pathogenic eukaryotic cells, in particular cancer cells. Cancer tissue / cells can be efficiently treated with freeze-dried polymer particles that are loaded with lanthanide compounds according to the present invention, thereby causing a dose-dependent reduction of cell growth or cell death.

**Best mode for carrying out the invention:**

The manufacture and applicability of the particulate system according to the invention is more fully explained in the following. The experimental data that support and demonstrate the present invention are shown in the accompanying Figures. Lanthanide compounds used in these experiments have a photon energy > 38 keV and a K absorption edge Z > 56.

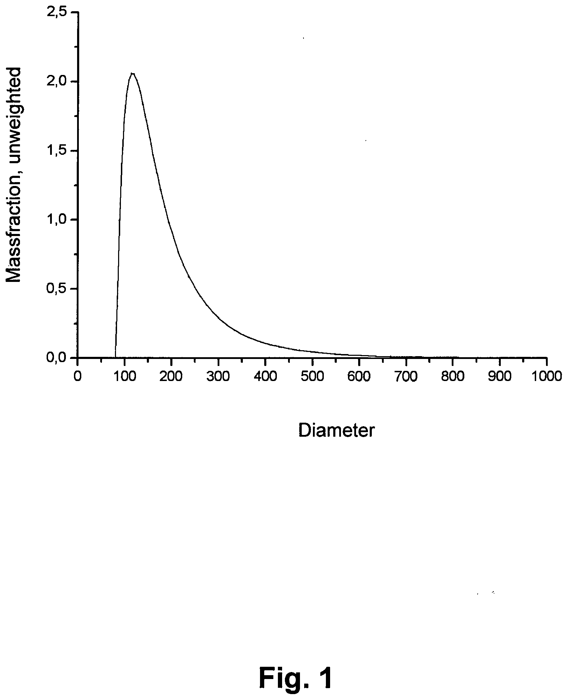

Fig. 1 shows the size distribution of nanoparticles produced by solvent evaporation. For purification and separation of the polymer particles, the suspension is centrifuged. The obtained supernatant is disregarded, the pellet washed for several times in water and then resuspended in a small volume of water. To increase purity, the centrifugation step is repeated twice. In order to obtain a freeze-dried powder of the particulate system of the invention, the pellet is re-suspended in a small amount of water and the whole suspension subsequently freeze-dried by addition of mannitol. According to Fig. 1, the size distribution peaks at a diameter of approximately 150 nm. The unweighted mass fraction at this diameter is around 2.0.

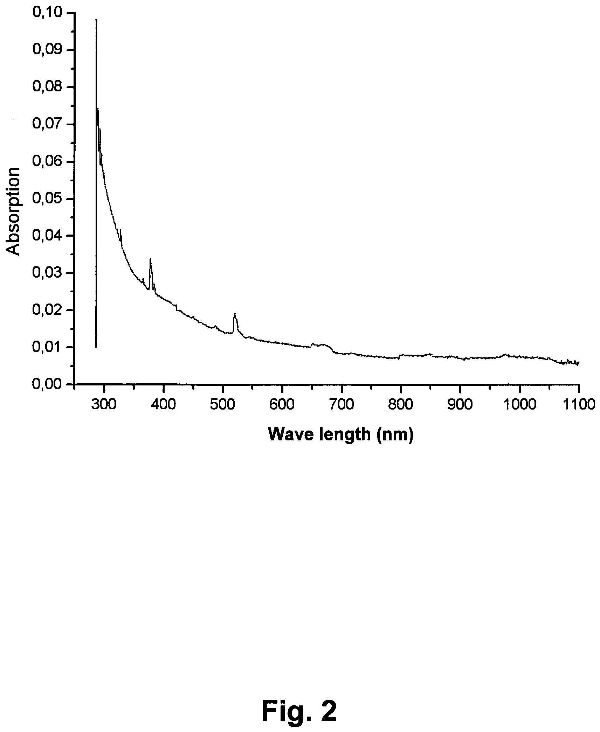

Fig. 2 shows the photometric detection of erbium in PLGA nanoparticles. The photometric determination of the erbium content is achieved by dissolving a defined amount of particles in DMF and comparison with a pure erbium-DMF solution of a known concentration. Experiments have been performed indicating that the large enrichment of the lanthanide compound in the polymer is due to intermolecular interactions. A mixture of polymer and erbium acetate in DMF was exposed to ultra filtration.

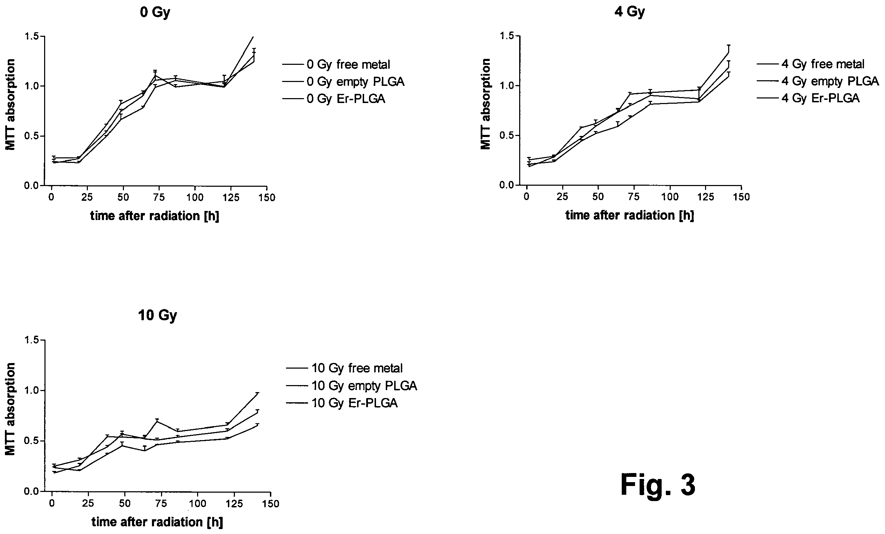

The pharmacological effect of the particulate system was investigated in cells of the lung carcinoma cell line A549. In order to determine a reduction of cell growth, A549 cells were seeded in 96 well plates and incubated for approximately 24 h before further treatments in order to reach nearly complete fixation of the cells to the bottom of the plates. The cells were then incubated together with polymer particles that were loaded with erbium for approximately 3 hours. Following exposing the cells with lanthanide particles, the cell culture plates were irradiated with different doses of irradiation. The proliferation of cells was determined by using a MTT growth assay in order to determine survival of irradiated tumor cells. The MTT assay allows the analysis of proliferation and determination of survival of cancer cells following irradiation and is based on a reduction of yellow water soluble tetra sodium salt to a purple water insoluble formazane dye by living cells. The cell proliferation over a period of 5 to 6 days following irradiation was analyzed. The results are presented in Fig. 3.

Fig. 3 shows growth curves of A549 cells following incubation with erbium nanoparticles and subsequent irradiation. Irradiation of particle treated cells was performed using a linear accelerator MD2. Already at very low doses of irradiation with 4 Gy, cell proliferation was significantly reduced using the particulate system according to the invention (ErPLGA) in comparison to the control (empty particles; empty PLGA). At higher doses, cell growth was further diminished. The experiments in Fig. 3 demonstrate that cell growth is reduced by more than 50 % after 75 h at a dose of 4 Gy using the lanthanide loaded polymer particles of the invention over the placebo control.

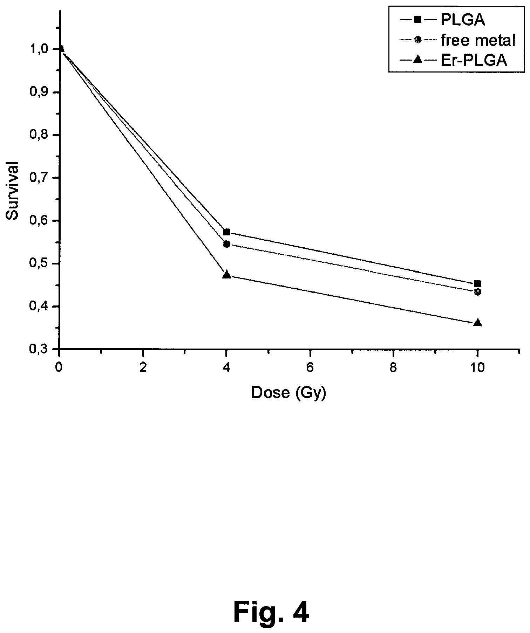

Fig. 4 shows the calculated survival of A549 cells after incubation with erbium, nanoparticles and subsequent irradiation.

Survival was calculated by using a mathematical approach in which cell survival is calculated using the following formula:

$$Survival = 2 \wedge -(t_{delay} / t_{doubling\ time})$$

T doubling time = time for cell doubling
T delay = time required to achieve a specific absorption value in the MTT test of the irradiated sample in comparison to the control.

Fig. 4 shows the dose-dependant survival of A549 cells. Survival of the untreated (non-irradiated) sample was set to 100 % at 0 Gy. The survival of the placebo control (empty PLGA) is reduced with increasing doses of irradiation. When free erbium as irradiation enhancer is added, cell survival was detected to be lower, whereas after incubation of the cells with erbium loaded particles, the reduction of cell growth is significantly higher.

In order to determine lanthanide-dependent absorption properties, A549 cells were incubated with different test samples and irradiated with variable irradiation doses. Irradiation with a monochromatic pattern above and below the k absorption profile of the lanthanide was applied.

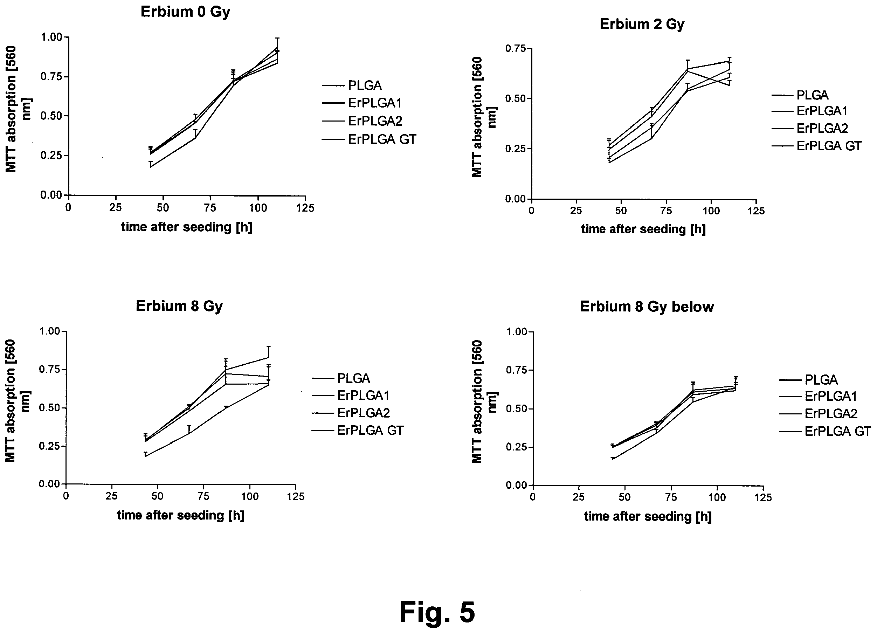

Fig. 5 shows cell growth of A549 cells after incubation with erbium nanoparticles and irradiation. PLGA = empty particle/placebo; ErPLGA1 = 0.6 mmol Er, ErPLGA2 = 1.2 mmol Er, ErPLGA GT = freeze-dried sample.

According to Fig. 5, cell growth is significantly diminished using ErPLGA particles in comparison with a control (empty particles). Surprisingly, the freeze-dried sample (ErPLGA GT) exhibits the strongest effect.

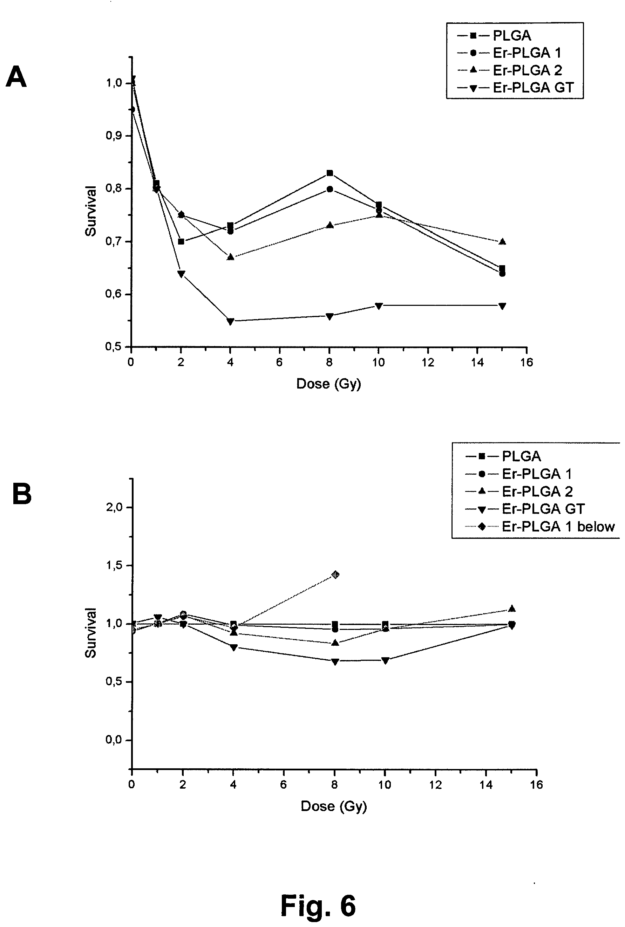

In Fig. 6, cell survival against increasing irradiation doses is demonstrated. In the experiments to Fig. 6A, survival of the placebo control of the non-irradiated sample was set to 100 %. In Fig. 6B, survival of the placebo control was set to 100 % for the respective irradiation dose.

According to Fig. 6, survival was significantly reduced using the particulate system of the invention as compared to the placebo control. The irradiation effect is dose-dependent, and peaks at 4 to 8 Gy. At higher irradiation doses, cell death is observed. The irradiation effect is dependent on the utilized lanthanide and the excitation frequency. Irradiation at wave lengths below the absorption profile of erbium, for instance, results in a survival similar to the control (Fig. 6B).

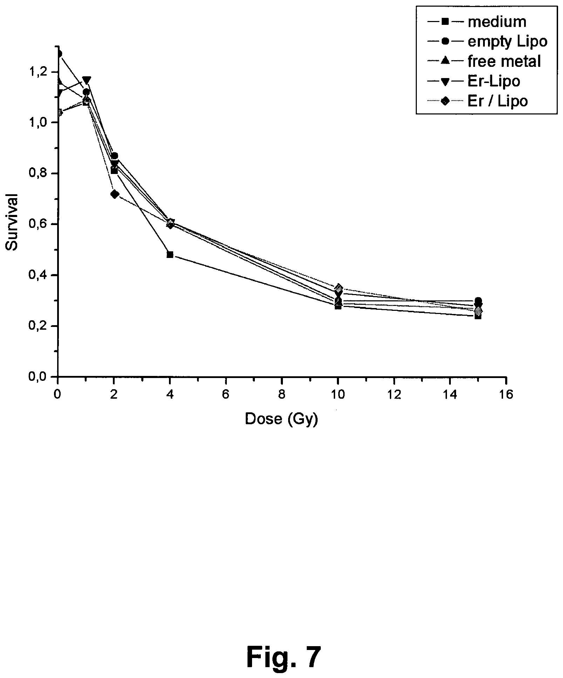

Fig. 7 is a comparative example that shows dose-dependent survival using liposomes as carrier for the lanthanide (erbium) instead of the solid polymer particle according to the invention. Fig. 7 clearly shows that there is no significant difference in survival after irradiation. Therefore, the particulate system of the invention has significant advantages over a liposome-based system.

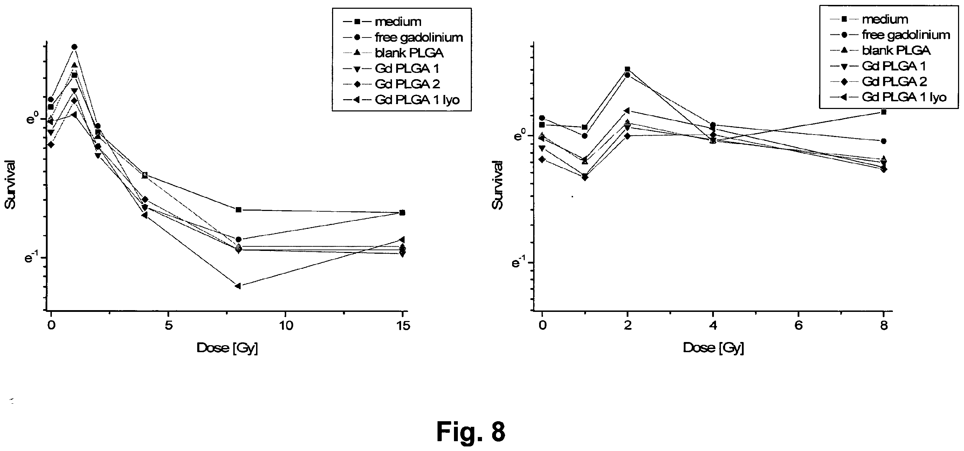

Fig. 8 shows the dose-dependent survival of A549 cells. The cells were treated with different media (pure medium, free gadolinium acetate, PLGA blank particles, Gd PLGA particles loaded with gadolinium acetate in freezed-dried form and in suspension). Fig. 8 demonstrates that freeze-dried particles have a significant higher efficiency as compared to non-freeze-dried particles (Gd PLGA 1, Gd PLGA 2, Gd PLGA 1 lyo).

Fig. 8 at the left side shows the results upon irradiation of the cells above the K threshold. Fig. 8 at the right side shows the specificity below the K threshold.

**Claims**

1. A particulate system for use in diminishing cell growth, in particular the growth of cancer cells, comprising one or more water soluble lanthanide compounds that are embedded in a solid biodegradable polymer particle, the polymer being selected from the group consisting of polycarbonic acids, polylactic acids, polyglycolic acids, polypeptides or combinations thereof, wherein the lanthanide compound of the polymer particle is a lanthanide acetate salt having a photon energy that is greater than 38 keV and a K absorption edge Z that is greater than 56.

2. The particulate system according to claim 1 for use in diminishing cell growth, in particular the growth of cancer cells according to claim 1, wherein the polymer particles loaded with one or more lanthanide compounds are provided in freeze-dried form.

3. The particulate system according to claim 1 or 2 for use in diminishing cell growth, in particular the growth of cancer cells according to claim 1, wherein the surface of the polymer particles loaded with one or more lanthanide compounds has been stabilized by detergents or stabilizers.

4. The particulate system according to any one of claims 1 to 3 for use in diminishing cell growth, in particular the growth of cancer cells according to claim 1, wherein the lanthanide compound is an acetate salt of lanthanum, cerium, praseodymium, neodymium, promethium, samarium, europium, gadolinium, erbium, dysprosium, holmium, thulium, yterbium, lutetium, scandium, yttrium, hafnium iridium, platin, gold, or bismuth.

5. The particulate system according to any one of claims 1 to 4 for use in diminishing cell growth, in particular the growth of cancer cells according to claim 1, wherein the polymer is selected from the group consisting of any optically active (D-/L-/DL-) forms of poly(glycolic acid) (PGA), poly(lactic acid) (PLA), poly (lactide-co-glycolide) copolymers (PLGA), polydioxanone (PDS), polyacrylates, polyketales, polycyanoacrylates, polyorthoesters, polyacetates, poly($\varepsilon$ caprolactone), polyphosphozene, polycarbonates, polypeptides, polyiminocarbonates, poly($\beta$-hydroxyester).

6. The particulate system according to any one of claims 1 to 5 for use in diminishing cell growth, in particular the growth of cancer cells according to claim 1, wherein the polymer has a free carboxylic acid end group, an ester terminated end group, or an alkyl ester end group.

7. The particulate system according to any one of claims 1 to 6 for use in diminishing cell growth, in particular the growth of cancer cells according to claim 1, wherein the polymer is poly(D,L-lactide-co-glycolide) with a free carboxylic acid end group.

8. The particulate system according to any one of claims 1 to 7 for use in diminishing cell growth, in particular the growth of cancer cells according to claim 1, wherein the lanthanide loaded polymer particles are obtained by solvent evaporation.

9. The particulate system according to any one of claims 1 to 8 for use in diminishing cell growth, in particular the growth of cancer cells according to claim 1, wherein the polymer particle further contains another irradiation enhancer and/or cytostatic.

10. An in-vitro method for diminishing cell growth, comprising the steps of exposing cells to a particulate system comprising one or more water soluble lanthanide compounds that are embedded in a solid biodegradable polymer particle, the polymer being selected from the group consisting of polycarbonic acids, polylactic acids, polyglycolic acids, polypeptides or combinations thereof, wherein the lanthanide compound of the polymer particle is a lanthanide acetate salt having a photon energy that is greater than 38 keV and a K absorption edge Z that is greater than 56, and exposing the cells that are treated with said particulate system to irradiation at a wave length that results in an excitation of the lanthanide compound(s).

**11.** The method according to claim 10, wherein the irradiation dose for treating the cells that were exposed to lanthanide loaded polymer particles with irradiation is at least 4 Gy.

**12.** A method for producing polymer particles, comprising one or more water soluble lanthanide acetate salts having a photon energy > 38 keV and a K absorption edge Z > 56 that are embedded in a solid biodegradable polymer particle, the polymer being selected from the group consisting of polycarbonic acids, polylactic acids, polyglycolic acids, polypeptides or combinations thereof by incubating the polymer with the lanthanide compound in a suitable solvent solution, emulsifying the polymer/lanthanide mixture and applying solvent evaporation to hardening the particles.

**13.** A pharmaceutical composition comprising one or more water soluble lanthanide compounds that are embedded in a solid biodegradable polymer particle, the polymer being selected from the group consisting of polycarbonic acids, polylactic acids, polyglycolic acids, polypeptides or combinations thereof, wherein the lanthanide compound of the polymer particle is a lanthanide acetate salt having a photon energy that is greater than 38 keV and a K absorption edge Z that is greater than 56 for use in the treatment of a pathological disease.

**14.** The pharmaceutical composition according to claim 13, wherein the pathological disease is a bacterial or fungal infection, or cancer.

**Patentansprüche**

**1.** Partikelsystem zur Verwendung zur Verminderung von Zellwachstum, insbesondere des Wachstums von Krebszellen, umfassend eine oder mehrere wasserlösliche Lanthanid-Verbindungen, die in einem festen biologisch abbaubaren Polymerpartikel eingebettet sind, wobei das Polymer ausgewählt ist aus der Gruppe bestehend aus Polycarbonsäuren, Polylactiden, Polyglycolsäuren, Polypeptiden oder Kombinationen davon, wobei die Lanthanid-Verbindung des Polymerpartikels ein Lanthanid-Acetat-Salz mit einer Photonenenergie von mehr als 38 keV und einer K-Absorptionskante Z größer als 56 ist.

**2.** Partikelsystem nach Anspruch 1 zur Verwendung zur Verminderung von Zellwachstum, insbesondere des Wachstums von Krebszellen gemäß Anspruch 1, wobei die Polymerpartikel, die mit einer oder mehreren Lanthanid-Verbindungen beladen sind, in gefriergetrockneter Form vorliegen.

**3.** Partikelsystem nach Anspruch 1 oder 2, wobei die Oberfläche der Polymerpartikel, die mit einer oder mehreren Lanthanid-Verbindungen beladen sind, durch Detergenzien oder Stabilisatoren stabilisiert ist.

**4.** Partikelsystem nach einem der Ansprüche 1 bis 3 zur Verwendung zur Verminderung von Zellwachstum, insbesondere des Wachstums von Krebszellen gemäß Anspruch 1, wobei die Lanthanid-Verbindung ein Acetatsalz von Lanthanum, Cerium, Praseodymium, Neodymium, Promethium, Samarium, Europium, Gadolinium, Erbium, Dysprosium, Holmium, Thulium, Yterbium, Lutetium, Scandium, Yttrium, Hafnium irridium, Platin, Gold oder Bismut ist.

**5.** Partikelsystem nach einem der Ansprüche 1 bis 4 zur Verwendung zur Verminderung von Zellwachstum, insbesondere des Wachstums von Krebszellen gemäß Anspruch 1, wobei das Polymer ausgewählt ist aus der Gruppe bestehend aus optisch aktiven (D-/L-/ DL-)Formen von Poly(Glycolsäure) (PGA), Poly(Lactid) (PLA), Poly (Lactid-Co-Glycolid) Copolymeren (PLGA), Polydioxanon (PDS), Polyacrylaten, Polyketalen, Polycyanoacrylaten, Polyorthoestern, Polyacetaten, Poly($\epsilon$ Caprolacton), Polyphosphozen, Polycarbonaten, Polypeptiden, Polyiminocarbonaten, Poly($\beta$-Hydroxyester).

**6.** Partikelsystem nach einem der Ansprüche 1 bis 5 zur Verwendung zur Verminderung von Zellwachstum, insbesondere des Wachstums von Krebszellen gemäß Anspruch 1, wobei das Polymer eine freie Carboxylsäure-Endgruppe, eine Ester-terminierte Endgruppe oder eine Alkylester-Endgruppe aufweist.

**7.** Partikelsystem nach einem der Ansprüche 1 bis 6 zur Verwendung zur Verminderung von Zellwachstum, insbesondere des Wachstums von Krebszellen gemäß Anspruch 1, wobei das Polymer Poly(D, L-Lactid-Co-Glycolid) mit einer freien Carboxylsäure-Endgruppe ist.

**8.** Partikelsystem nach einem der Ansprüche 1 bis 7 zur Verwendung zur Verminderung von Zellwachstum, insbesondere des Wachstums von Krebszellen gemäß Anspruch 1, wobei das mit Polymerpartikeln geladene Lanthanid durch Lösungsmittelevaporation gewonnen wurde.

**9.** Partikelsystem nach einem der Ansprüche 1 bis 8 zur Verwendung zur Verminderung von Zellwachstum, insbesondere des Wachstums von Krebszellen gemäß Anspruch 1, wobei das Polymerpartikel weiter einen anderen Strahlungsverstärker und/oder Cytostatikum enthält.

**10.** Verfahren zur Verminderung von Zellwachstum, umfassend die Schritte, bei denen Zellen einem Partikelsystem ausgesetzt werden, das eine oder mehrere wasserlösliche Lanthanid-Verbindungen umfasst, die in einem festen bioabbaubaren Polymerpartikel eingebettet sind, wobei das Polymer ausgewählt aus der Gruppe bestehend aus Polycarbonsäuren, Polylactiden, Polyglycolsäuren, Polypeptiden oder Kombinationen davon, wobei die Lanthanid-Verbindung des Polymerpartikels ein Lanthanid-Acetat-Salz mit einer Photonenenergie von mehr als 38 keV und einer K-Absorptionskante Z größer als 56 ist, und bei dem die mit dem Partikelsystem behandelten Zellen einer Strahlung ausgesetzt werden, bei einer Wellenlänge, die eine Anregung der Lanthanid-Verbindung(en) bewirkt.

11. Verfahren nach Anspruch 10, wobei die Strahlungsdosis zur Behandlung der Zellen, die mit Lanthanid-beladenen Poylmerpartikeln einer Strahlung ausgesetzt wurden, wenigsten 4 Gy beträgt.

12. Verfahren zur Herstellung von Polymerpartikeln, die ein oder mehrere wasserlösliche Lanthanid-Acetat-Salze mit einer Photonenenergie > 38 keV und einer K Absorptionskante Z > 56 umfassen, die in einem festen bioabbaubaren Polymerpartikel eingebettet sind, wobei das Polymer ausgewählt ist aus der Gruppe bestehend aus Polycarbonsäuren, Polylactiden, Polyglycolsäuren, Polypeptiden oder Kombinationen davon, durch Inkubieren des Polymers mit der Lanthanid-Verbindung in einer geeigneten Lösungsmittellösung, Emulgieren des Polymer-/ Lanthanid-Gemisches und Anwendung einer Lösungsmittelevaporation zur Verfestigung der Partikel.

13. Pharmazeutische Zusammensetzung, umfassend eine oder mehrere wasserlösliche Lanthanid-Verbindungen, die in einem festen bioabbaubaren Polymerpartikel eingebettet sind, wobei das Polymer ausgewählt ist aus der Gruppe bestehend aus Polycarbonsäuren, Polylactiden, Polyglycolsäuren, Polypeptiden oder Kombinationen davon, wobei die Lanthanid-Verbindung des Polymerpartikels ein Lanthanid-Acetat-Salz mit einer Photonenenergie von mehr als 38 keV und einer K-Absorptionskante Z größer als 56 ist, zur Verwendung für die Behandlung einer pathologischen Erkrankung.

14. Pharmazeutische Zusammensetzung nach Anspruch 13, wobei die pathologische Erkrankung eine bakterielle Infektion, eine Pilzinfektion oder Krebs ist.

**Revendications**

1. Système à particules destiné à être utilisé pour diminuer la croissance cellulaire, en particulier la croissance de cellules cancéreuses, comprenant un ou plusieurs composés de lanthanide solubles dans l'eau qui sont intégrés dans une particule de polymère biodégradable solide, le polymère étant sélectionné parmi le groupe constitué d'acides polycarboniques, d'acides polylactiques, d'acides polyglycoliques, de polypeptides, ou de combinaisons de ceux-ci, où le composé de lanthanide de la particule de polymère est un sel d'acétate de lanthanide ayant une énergie de photons qui est supérieure à 38 keV et une discontinuité d'absorption K Z qui est supérieure à 56.

2. Système à particules selon la revendication 1, destiné à être utilisé pour diminuer la croissance cellulaire, en particulier la croissance de cellules cancéreuses selon la revendication 1, où les particules de polymère chargées avec un ou plusieurs composés de lanthanide sont fournies sous une forme lyophilisée.

3. Système à particules selon la revendication 1 ou 2, destiné à être utilisé pour diminuer la croissance cellulaire, en particulier la croissance de cellules cancéreuses selon la revendication 1, où la surface des particules de polymère chargées avec un ou plusieurs composés de lanthanide a été stabilisée à l'aide de détergents ou d'agents stabilisants.

4. Système à particules selon l'une quelconque des revendications 1 à 3, destiné à être utilisé pour diminuer la croissance cellulaire, en particulier la croissance de cellules cancéreuses selon la revendication 1, où le composé de lanthanide est un sel d'acétate de lanthane, de cérium, de praséodyme, de néodyme, de prométhium, de samarium, d'europium, de gadolinium, d'erbium, de dysprosium, d'holmium, de thulium, d'ytterbium, de lutécium, de scandium, d'yttrium, d'hafnium, d'iridium, de platine, d'or ou de bismuth.

5. Système à particules selon l'une quelconque des revendications 1 à 4, destiné à être utilisé pour diminuer la croissance cellulaire, en particulier la croissance de cellules cancéreuses selon la revendication 1, où le polymère est sélectionné parmi le groupe constitué de toutes les formes optiquement actives (formes D/L/DL) de poly(acide glycolique) (PGA), de poly(acide lactique) (PLA), de copolymères poly(lactide-co-glycolide) (PLGA), de polydioxanone (PDS), de polyacrylates, de polyacétals, de polycyanoacrylates, de polyorthoesters, de polyacétates, de poly($\varepsilon$-caprolactone), de polyphosphazène, de polycarbonates, de polypetides, de polyiminocarbonates, de poly($\beta$-hydroxyester).

6. Système à particules selon l'une quelconque des revendications 1 à 5, destiné à être utilisé pour diminuer la croissance cellulaire, en particulier la croissance de cellules cancéreuses selon la revendication 1, où le polymère présente un groupement terminal acide carboxylique libre, un groupement terminal à terminaison ester, ou un groupement terminal ester d'alkyle.

7. Système à particules selon l'une quelconque des revendications 1 à 6, destiné à être utilisé pour diminuer la croissance cellulaire, en particulier la croissance de cellules cancéreuses selon la revendication 1, où le polymère est le poly(D,L-lactide-co-glycolide) avec un groupement terminal acide carboxylique libre.

8. Système à particules selon l'une quelconque des revendications 1 à 7, destiné à être utilisé pour diminuer la croissance cellulaire, en particulier la croissance de cellules cancéreuses selon la revendication 1, où les particules de polymère chargées en lanthanide sont obtenues par évaporation de solvant.

9. Système à particules selon l'une quelconque des revendications 1 à 8, destiné à être utilisé pour diminuer la croissance cellulaire, en particulier la croissance de cellules cancéreuses selon la revendication 1, où la particule de polymère contient en outre un autre améliorateur d'irradiation et/ou un cytostatique.

10. Procédé in vitro de diminution de la croissance cellulaire, comprenant les étapes consistant à exposer des cellules à un système à particules comprenant un ou plusieurs composés de lanthanide solubles dans l'eau qui sont intégrés dans une particule de polymère biodégradable solide, le polymère étant sélectionné parmi le groupe constitué d'acides polycarboniques, d'acides polylactiques, d'acides polyglycoliques, de polypeptides, ou de combinaisons de ceux-ci, où le composé de lanthanide de la particule de polymère est un sel d'acétate de lanthanide ayant une énergie de photons qui est supérieure à 38 keV et une discontinuité d'absorption K Z qui est supérieure à 56, et à exposer les cellules qui sont traitées avec ledit système à particules à une irradiation à une certaine longueur d'onde qui provoque une excitation du ou des composés de lanthanide.

11. Procédé selon la revendication 10, dans lequel la dose d'irradiation pour traiter les cellules qui ont été exposées aux particules de polymère chargées en lanthanide avec irradiation est au moins de 4 Gy.

12. Procédé de production de particules de polymère, comprenant un ou plusieurs sels d'acétate de lanthanide solubles dans l'eau ayant une énergie de photons > 38 keV et une discontinuité d'absorption K Z > 56 qui sont intégrés dans une particule de polymère biodégradable solide, le polymère étant sélectionné parmi le groupe constitué d'acides polycarboniques, d'acides polylactiques, d'acides polyglycoliques, de polypeptides, ou de combinaisons de ceux-ci, par incubation du polymère avec le composé de lanthanide dans une solution de solvant appropriée, par émulsification du mélange polymère/lanthanide et par réalisation d'une évaporation de solvant pour un durcissement des particules.

13. Composition pharmaceutique comprenant un ou plusieurs composés de lanthanide solubles dans l'eau qui sont intégrés dans une particule de polymère biodégradable solide, le polymère étant sélectionné parmi le groupe constitué d'acides polycarboniques, d'acides polylactiques, d'acides polyglycoliques, de polypeptides, ou de combinaisons de ceux-ci, où le composé de lanthanide de la particule de polymère est un sel d'acétate de lanthanide ayant une énergie de photons qui est supérieure à 38 keV et une discontinuité d'absorption K Z qui est supérieure à 56, destinée à être utilisée dans le traitement d'une maladie pathologique.

14. Composition pharmaceutique selon la revendication 13, où la maladie pathologique est une infection bactérienne ou fongique, ou un cancer.

**Fig. 1**

# Fig. 2

Fig. 3

Fig. 4

Fig. 5

**Fig. 6**

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6770020 B2
- US 5888997 A
- EP 01292298 A
- US 6040432 A
- WO 2009121631 A2

### Non-patent literature cited in the description

- **CARAVAN P. ; ELLISON J.J. ; MCMURRY T.J. ; LAUFFER R.B.** Gadolinium(III) Cheleates as MRI Contrast Agents: Structure, Dynamics, and Applications. *Chem. Rev.,* 1999, vol. 99, 2293-2352
- **WIENER E.C. ; KONDA S. ; SHADRON A. ; BRECHBIEL M. ; GANSOW O.** Targeting dendrimer-chelates to tumors and tumor cells expressing the high-affinity folate receptor. *Invest. Radiol.,* 1997, vol. 32, 748-54
- **T. NAWROTH et al.** Synchrotron Irradiation in Material Sciences. *SRMS 4, Conference,* 23 August 2004